(19) Europäisches Patentamt / European Patent Office / Office européen des brevets

(11) **EP 4 516 238 A1**

(12) **EUROPEAN PATENT APPLICATION**

(43) Date of publication:
05.03.2025 Bulletin 2025/10

(21) Application number: 23195358.9

(22) Date of filing: 05.09.2023

(51) International Patent Classification (IPC):
*A61B 8/08* (2006.01)   *A61B 8/00* (2006.01)

(52) Cooperative Patent Classification (CPC):
A61B 8/0883; A61B 8/463; A61B 8/5269;
A61B 8/5284

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 01.09.2023 US 202363530088 P

(71) Applicant: Koninklijke Philips N.V.
5656 AG Eindhoven (NL)

(72) Inventors:
• MUKADDIM, Rashid Al
  Eindhoven (NL)
• ERKAMP, Ramon Quido
  Eindhoven (NL)
• BHARAT, Shyam
  5656AG Eindhoven (NL)

(74) Representative: Philips Intellectual Property & Standards
High Tech Campus 52
5656 AG Eindhoven (NL)

(54) **REMOVAL OF MOTION-CORRUPTED ULTRASOND IMAGES FOR OPTIMAL MULTIBEAT CARDIAC QUANTIFICATION**

(57)    An ultrasound imaging system includes a processor for communication with a display, a transducer array of a handheld ultrasound probe, and a memory. The processor receives multiple ultrasound images of a patient anatomy and identifies one or more motion corrupted ultrasound images. The processor then extracts the one or more motion corrupted ultrasound images to form temporal data blocks of the remaining ultrasound images. The processor then determines whether the temporal data blocks each correspond to at least one periodic cycle and calculates a metric based on each temporal data block. An ultrasound image, the metric, a status indicator corresponding to the one of the temporal data blocks, and a status indicator corresponding to the extracted motion corrupted ultrasound images are then displayed.

Fig. 6

**Description**

FIELD OF THE INVENTION

The present disclosure relates generally to the field of ultrasound imaging. In particular, the present disclosure relates to systems and methods for identifying and extracting motion-corrupted ultrasound images and calculating quantification metrics based on the remaining ultrasound images.

BACKGROUND OF THE INVENTION

Physicians use many different medical diagnostic systems and tools to monitor a subject's health and diagnose and treat medical conditions. Ultrasound imaging systems are widely used for medical imaging and measurement. The ultrasound transducer probe may include an array of ultrasound transducer elements that transmit acoustic waves into a subject's body and record acoustic waves reflected and backscattered from the internal anatomical structures within the subject's body, which may include tissues, blood vessels, and internal organs. The transmission and reception of acoustic waves, along with various beamforming and processing techniques, create an image of the subject's internal anatomical structures.

Ultrasound imaging is a safe, useful, and in some applications, non-invasive tool for diagnostic examination, interventions, and/or treatment. Ultrasound imaging can be used to diagnose a wide variety of medical conditions. Ultrasound imaging may be used to image motion of a patient's heart and quantify performance. However, obtaining accurate measurements can be challenging, as aperiodic or non-cardiac motion can cause interference that can lead to errors in the data. Aperiodic motion can arise from several sources, including movement of the probe during acquisition, or movement of the patient's body during the acquisition process, such as due to breathing or other motion. This aperiodic or non-cardiac motion may cause drift in the measurements leading to inaccurate metrics. Inaccuracies in physiological signal measurements can have significant consequences for patient care, as incorrect diagnoses or treatment plans may be developed based on inaccurate data.

SUMMARY OF THE INVENTION

Aspects of the present disclosure are systems, devices, and methods for optimal multibeat cardiac quantification. Aspects of the present disclosure advantageously identify and extract motion corrupted images from a set of ultrasound images received during an imaging procedure. The system then calculates quantification metrics only on the non-extracted images to increase the accuracy of the results. Aspects of the present disclosure may assist novice users or users in non-ideal procedure settings to calculate accurate results based on data received even if the data includes motion-corrupted ultrasound images.

In some aspects, a processor of the system receives multiple ultrasound images during or after an imaging procedure. The processor then identifies motion-corrupted images and removes them from the set. The processor then analyzes the remaining ultrasound images and forms temporal data blocks of the ultrasound images. A temporal data block may be a subset of the ultrasound images that are not motion corrupted and correspond to at least one cardiac beat cycle. Ultrasound images that are not motion-corrupted but are within the same cardiac beat cycle as motion-corrupted ultrasound images may also be extracted. The processor system then calculates metrics based on the temporal data blocks including the remaining ultrasound images. As a result, the metrics calculated are based only on non-motion-corrupted ultrasound images, increasing the accuracy of the metrics.

In an exemplary aspect, an ultrasound imaging system is provided. The ultrasound imaging system comprises a processor configured for communication with a display, a transducer array of a handheld ultrasound probe, and a memory, wherein the processor is configured to: receive a plurality of ultrasound images corresponding to a view of a patient anatomy; identify one or more motion corrupted ultrasound images of the plurality of ultrasound images; extract the one or more motion corrupted ultrasound images from the plurality of ultrasound images; form a first temporal data block, the first temporal data block comprising at least some (or even all) of the remaining ultrasound images from the plurality of ultrasound images; determine whether the first temporal data block corresponds to at least one periodic cycle; calculate a metric based on the first temporal data block; and generate an output for the display, the output comprising: an ultrasound image of the plurality of ultrasound images; the metric; a first status indicator corresponding to the first temporal data block; and a second status indicator corresponding to the extracted motion corrupted ultrasound images.

In some aspects, the processor is further configured to determine an image quality of the plurality of ultrasound images.

In some aspects, the processor is further configured to identify the one or more motion corrupted images by identifying and tracking a feature within the plurality of ultrasound images.

In some aspects, to track the feature, the processor is configured to: determine a change in location of the feature within each ultrasound image of the plurality of ultrasound images; and identify an ultrasound image in which the change in location of the feature exceeds a threshold.

In some aspects, the processor is further configured to identify the one or more motion corrupted images based on one or more of traditional image processing, RF data peak hopping, AI image processing, AI processing on RF data, or AI processing on ultrasound B-mode data.

In some aspects, the metric comprises at least one of an end diastolic volume (EDV), end systolic volume (ESV), stroke volume (SV), ejection fraction (EF), global longitudinal strain (GLS), or cardiac output (CO).

In some aspects, the periodic cycle comprises a cardiac beat cycle. The periodic cycle may advantageously comprise one or more cardiac beat cycles.

In some aspects, the metric comprises an average, such as an average of data of the first temporal data block. For example, when the periodic cycle comprises a plurality of cardiac beat cycles, the metric may adavantageoulsy comprise an average over some or even all the cardiac beat cycles in said plurality.

In some aspects, the first temporal data block comprises a first subset of the remaining ultrasound images from the plurality of ultrasound images; and the processor is further configured to form a second temporal data block, the second temporal data block comprising a second subset of the remaining ultrasound images from the plurality of ultrasound images, wherein the first temporal data block and the second temporal data block are temporally spaced by the motion corrupted ultrasound images. In some of these aspects, the metric comprises an average of data from the first temporal data block and the second temporal data block.

In some aspects, the processor is further configured to receive an input from the user accepting the metric. In some aspects, the processor is further configured to receive an input from the user rejecting the metric.

In some aspects, the processor is further configured to receive a first user input identifying a first periodic cycle of the first temporal data block. In some of these aspects, the processor is further configured to discard a subset of ultrasound images corresponding to the first periodic cycle of the first temporal data block based on a second user input.

In some aspects, the ultrasound image further comprises an outline representative of a structure of the patient anatomy and corresponding to the metric. In particular, in some aspects, to generate the output, the processor is further configured to generate said outline on the ultrasound image.

In some aspects, the ultrasound imaging system further comprises: the display, a handheld ultrasound probe comprising the transducer array, and the memory.

In an exemplary aspect, an ultrasound system for measuring performance of a cardiac system of a patient is provided. The ultrasound system includes a handheld ultrasound probe comprising a transducer array; a display; a memory; and a processor configured for communication with the transducer array, the display, and the memory, wherein the processor is configured to: control the transducer array to obtain a plurality of ultrasound images corresponding to a view of a left ventricle of a heart of a patient; identify one or more motion corrupted ultrasound images of the plurality of ultrasound images; extract the one or more motion corrupted ultrasound images from the plurality of ultrasound images; form a first temporal data block, the first temporal data block comprising at least a subset of the remaining ultrasound images from the plurality of ultrasound images; determine whether the first temporal data block corresponds to at least one cardiac cycle; calculate a metric based on the first temporal data block; and generate an output for the display, the output comprising: an ultrasound image of the plurality of ultrasound images; the metric; a first status indicator corresponding to the first temporal data block; and a second status indicator corresponding to the extracted motion corrupted ultrasound images.

In an exemplary aspect, a processor circuit for extracting motion corrupted ultrasound images from a set of acquired ultrasound images and performing a quantification based on the acquired set is provided. The processor circuit comprises a processor configured to: receive a plurality of ultrasound images corresponding to a view of a patient anatomy; identify one or more motion corrupted ultrasound images of the plurality of ultrasound images; extract the one or more motion corrupted ultrasound images from the plurality of ultrasound images; form a first temporal data block, the first temporal data block comprising at least some (or even all) of the remaining ultrasound images from the plurality of ultrasound images; determine whether the first temporal data block corresponds to at least one periodic cycle; calculate a metric based on the first temporal data block; and generate an output, the output comprising: an ultrasound image of the plurality of ultrasound images; the metric; a first status indicator corresponding to the first temporal data block; and a second status indicator corresponding to the extracted motion corrupted ultrasound images.

In some aspects, the processor circuit further comprises a memory and a communication module, wherein the processor, the memory and the communication module are configured for communication with each other, and wherein the communication module is further configured for communication with a display and a transducer array of a handheld ultrasound probe.

In ane exemplary aspect, an ultrasound imaging system comprises: a handheld ultrasound probe comprising a transducer array; a display, and a processor circuit as defined above.

In an exemplary aspect, a method for extracting motion corrupted ultrasound images from a set of acquired ultrasound images and performing a quantification based on the acquired set is provided. The method comprises:

receiving a plurality of ultrasound images from an ultrasound imaging system, the plurality of ultrasound images corresponding to a view of a patient anatomy; identifying one or more motion corrupted ultrasound images of the plurality of ultrasound images; extracting the one or more motion corrupted ultrasound images from the plurality of ultrasound images; forming a first temporal data block, the first temporal data block comprising at least a subset of the remaining ultrasound images from the plurality of ultrasound images; determining whether the first temporal data block corresponds to at least one periodic cycle; calculating a metric based on the first temporal data block; and generating an output, the output comprsing: an ultrasound image of the plurality of ultrasound images; the metric; a first status indicator corresponding to the first temporal data block; and a second status indicator corresponding to the extracted motion corrupted ultrasound images.

In an exemplary aspect, a computer program product is provided. The computer program product comprises computer-readable instructions which, when executed by a processor, cause the processor to carry out the method as defined above.

Additional aspects, features, and advantages of the present disclosure will become apparent from the following detailed description.

BRIEF DESCRIPTION OF THE DRAWINGS

Illustrative aspects of the present disclosure will be described with reference to the accompanying drawings, of which:

Fig. 1 is a schematic diagram of an ultrasound imaging system, according to aspects of the present disclosure.
Fig. 2 is a schematic diagram of a processor circuit, according to aspects of the present disclosure.
Fig. 3 is a graphical representation of a patient metric based on acquired ultrasound image data, according to aspects of the present disclosure.
Fig. 4 is a graphical representation of a patient metric based on acquired ultrasound image data, according to aspects of the present disclosure.
Fig. 5 is a flow diagram of a method of extracting motion corrupted ultrasound images from a set of acquired ultrasound images and performing a quantification based on the acquired set, according to aspects of the present disclosure.
Fig. 6 is a graphical representation of a method of extracting motion corrupted ultrasound images from a set of acquired ultrasound images and performing a quantification based on the acquired set, according to aspects of the present disclosure.
Fig. 7 is a graphical representation of a graphical user interface, according to aspects of the present disclosure.
Fig. 8 is a graphical representation of a graphical user interface, according to aspects of the present disclosure.
Fig. 9 is a graphical representation of a graphical user interface, according to aspects of the present disclosure.
Fig. 10 is a graphical representation of a method of extracting motion corrupted ultrasound images, according to aspects of the present disclosure.
Fig. 11 is a graphical representation of a method of identifying motion corrupted ultrasound images, according to aspects of the present disclosure.

DETAILED DESCRIPTION OF EMBODIMENTS

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

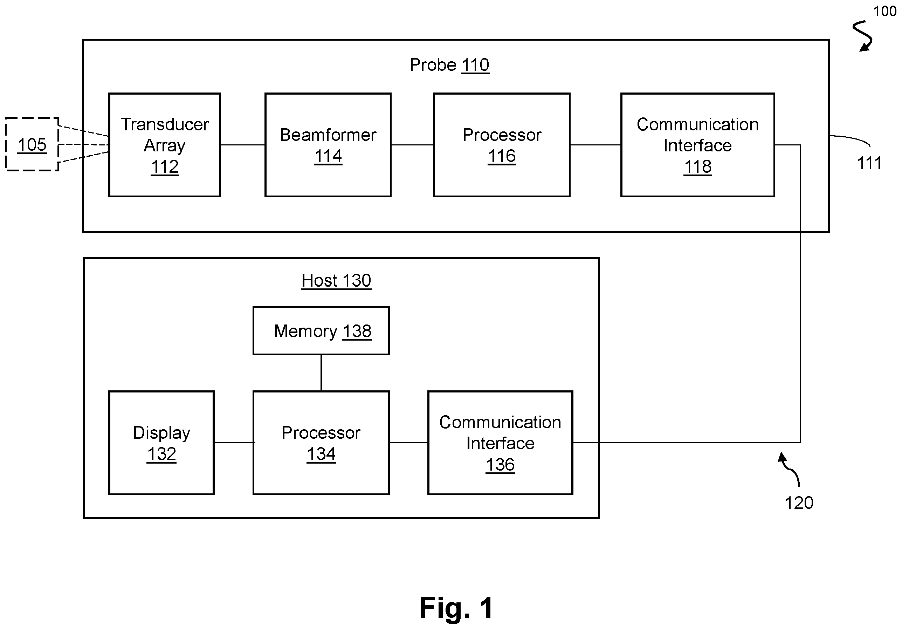

Fig. 1 is a schematic diagram of an ultrasound imaging system 100, according to aspects of the present disclosure. The system 100 is used for scanning an area or volume of a subject's body. A subject may include a patient of an ultrasound imaging procedure, or any other person, or any suitable living or non-living organism or structure. The system 100 includes an ultrasound imaging probe 110 in communication with a host 130 over a communication interface or link 120. The probe 110 may include a transducer array 112, a beamformer 114, a processor 116, and a communication interface 118. The host 130 may include a display 132, a processor 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor 116 based on the response of the received ultrasound echo signals. The beamformer 114 may include multiple stages of beamforming. The beamforming can reduce the number of signal lines for coupling to the processor 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor 134. The display 132 may be a monitor or any suitable display. The display 132 is configured to display the ultrasound images, image videos, and/or any imaging information of the object 105.

The system 100 may be used to assist a sonographer in performing an ultrasound scan. The scan may be performed in a at a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound system 100 may then analyze the ultrasound image to identify various parameters associated with the acquisition of the image such as the scan window, the probe orientation, the subject position, and/or other parameters. The system 100 may then store the image and these associated parameters in the memory 138. At a subsequent imaging procedure, the system 100 may retrieve the previously acquired ultrasound image and associated parameters for display to a user which may be used to guide the user of the system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

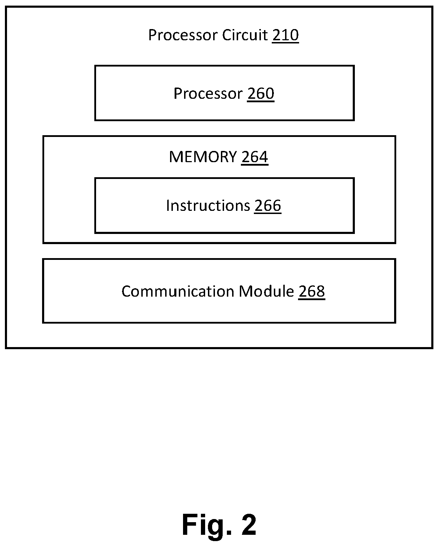

Fig. 2 is a schematic diagram of a processor circuit, according to aspects of the present disclosure. One or more processor circuits can be configured to carry out the operations described herein. The processor circuit 210 may be implemented in the probe 110, the host system 130 of Fig. 1, or any other suitable location. For example, the processor 116 of the probe 110 can be part of the processor circuit 210. For example, the processor 134 and/or the memory 138 can be part of the processor circuit 210. In an example, the processor circuit 210 may be in communication with the transducer array 112, beamformer 114, communication interface 122, communication interface 136, and/or the display 132, as well as any other suitable component or circuit within ultrasound system 100. As shown, the processor circuit 210 may include a processor 260, a memory 264, and a communication module 268. These elements may be in direct or indirect communication with each other, for example via one or more buses.

The processor 260 may include a CPU, a GPU, a DSP, an application-specific integrated circuit (ASIC), a controller, an FPGA, another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration. The processor 260 may also include an analysis module as will be discussed in more detail hereafter. The analysis module may implement various machine learning algorithms and may be a hardware or a software implementation. The processor 260 may additionally include a preprocessor in either hardware or software implementation. The processor 260 may execute various instructions, including instructions stored on a non-transitory computer readable medium, such as the memory 264.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In some instances, the memory 264 includes a non-transitory computer-readable medium. The memory 264 may store instructions 266. The instructions 266 may include instructions that, when executed by the processor 260, cause the processor 260 to perform the operations described herein with reference to the probe 110 and/or the host 130 (Fig. 1). Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 266 may include various aspects of a preprocessor, machine learning algorithm, convolutional neural network (CNN) or various other instructions or code. In some aspect, the memory 264 may be or include a non-transitory computer readable medium.

The communication module 268 can include any electronic circuitry and/or logic circuitry to facilitate direct or indirect communication of data between the processor circuit 210, the probe 110, and/or the host 130. In that regard, the communication module 268 can be an input/output (I/O) device. In some instances, the communication module 268 facilitates direct or indirect communication between various elements of the processor circuit 210 and/or the probe 110 (Fig. 1) and/or the host 130 (Fig. 1).

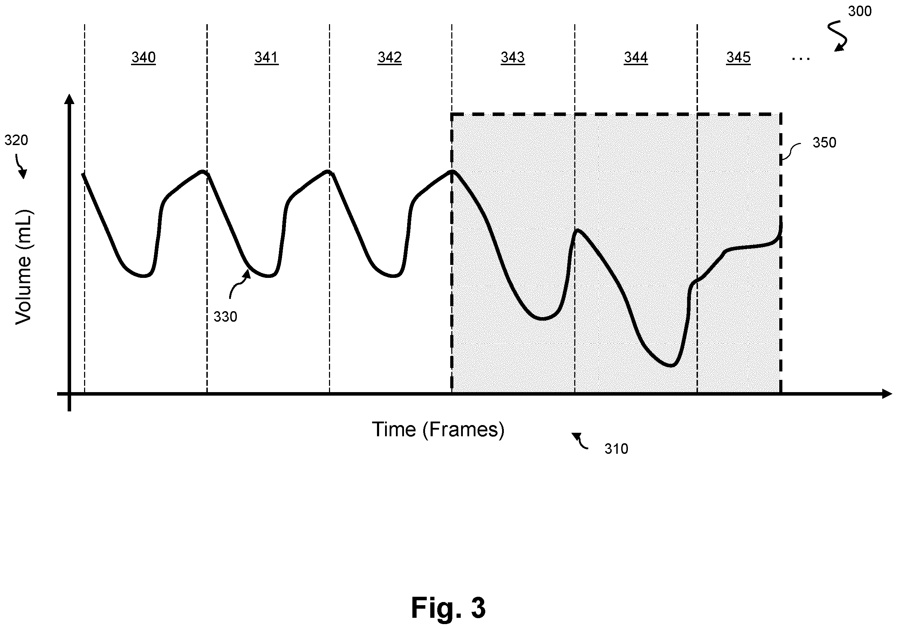

Fig. 3 is a graphical representation of a patient metric measured over time based on acquired ultrasound image data, according to aspects of the present disclosure. Fig. 3 includes a plot 300 that shows a dataset 330 representing ultrasound data acquired over time. The plot 300 includes an x-axis 310 corresponding to time, which can be in any suitable units such as frames or seconds, and a y-axis 320 corresponding to volume, which can be in any suitable units such as milliliters, cubic centimeters, or any other suitable unit of volume.

The dataset 330 shows multiple periodic cycles, which can be identified by several regions 340, 341, 342, 343, 344, and 345. The plot 300 may include any suitable number of cycles, and the cycles may correspond to a particular structure or organ being imaged. For example, a cycle may correspond to a cardiac cycle, where the heart contracts and expands to pump blood through the body. In other aspects, the region of interest being imaged may be any other organ or structure in the patient anatomy that also shows cyclical motion, such as lungs, structures of the gastrointestinal tract, or any other features of a patient anatomy. The plot 300 may include any suitable number of cycles.

The regions 340, 341, and 342 may correspond to data obtained by the ultrasound transducer while the transducer and the patient are stationary. In contrast, the regions 343, 344, and 345 may correspond to data obtained by the ultrasound transducer while the transducer or the patient is moving. As a result, the regions 343, 344, and 345 may correspond to motion-corrupted ultrasound images that may contain artifacts or distortions.

A graphical element 350 may be used to identify the regions of the plot 300 corresponding to motion-corrupted images. The graphical element 350 may be any suitable visual marker, such as a color or a shading pattern, that distinguishes the regions 343, 344, and 345 from the regions 340, 341, and 342. The graphical element 350 may be applied automatically by a computer program or manually by a user, depending on the particular application.

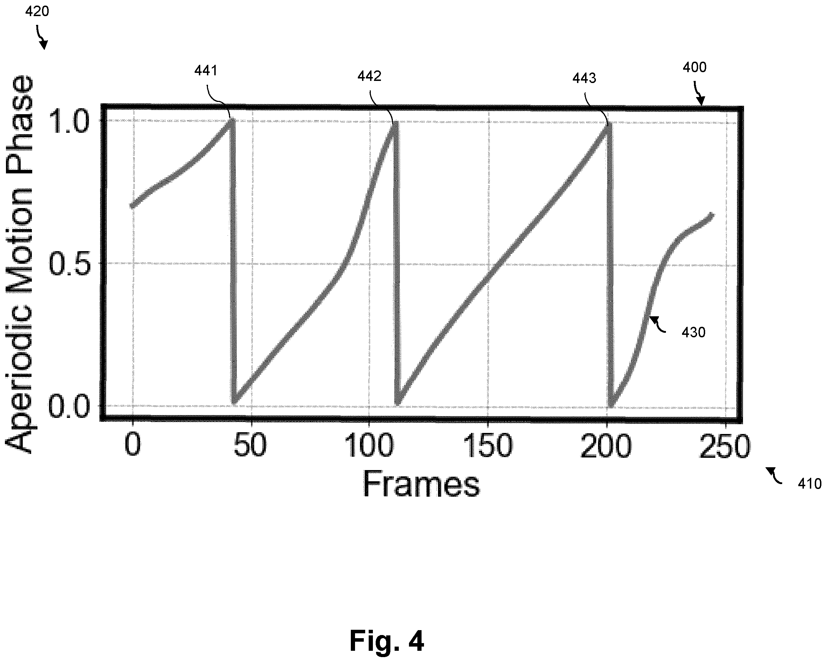

Fig. 4 is a graphical representation of a patient metric based on acquired ultrasound image data, according to aspects of the present disclosure. Fig. 4 includes a plot 400 that displays a dataset 430 of ultrasound data acquired over time. The plot 400 includes an x-axis 410 representing time, which can be in any suitable units such as frames or seconds, and a y-axis 420 representing an aperiodic motion phase.

The dataset 430 shows multiple peaks 441, 442, and 443. Each peak corresponds to a point in time where the motion of the ultrasound transducer or the patient exceeds a threshold such that the phase shifts from one extreme to the other. These peaks are caused by extreme aperiodic motion, such as sudden jerks or movements, and the severity and duration of the motion may correspond to the number of peaks present in the plot 400. In some aspects, the presence of motion peaks in the ultrasound data may indicate that the resulting images are motion-corrupted and may contain artifacts or distortions.

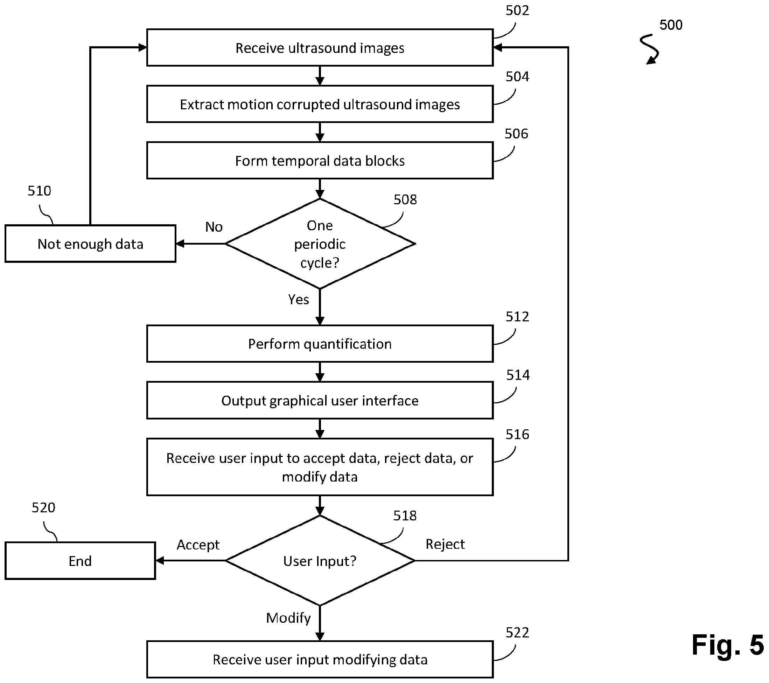

Fig. 5 is a flow diagram of a method of extracting motion corrupted ultrasound images from a set of acquired ultrasound images and performing a quantification based on the acquired set, according to aspects of the present disclosure. As illustrated, the method 500 includes a number of enumerated steps, but aspects of the method 500 may include additional steps before, after, or in between the enumerated steps. In some aspects, one or more of the enumerated steps may be omitted, performed in a different order, or performed concurrently. The steps of the method 500 can be carried out by any suitable component within the system 100 and all steps need not be carried out by the same component. In some aspects, one or more steps of the method 500 can be performed by, or at the direction of, a processor, including, e.g., the processor 116 (Fig. 1), the processor 134 (Fig. 1), the processor 260 (Fig. 2) or any other suitable component.

Step 502 of the method 500 includes receiving ultrasound images. The ultrasound images may be obtained from any suitable ultrasound imaging system, such as a two-dimensional (2D) or three-dimensional (3D) ultrasound imaging system. In some aspects, the ultrasound images may be obtained using a probe that is placed on the surface of the patient's body. The ultrasound images may include any suitable number of frames and may be obtained at any suitable frame rate.

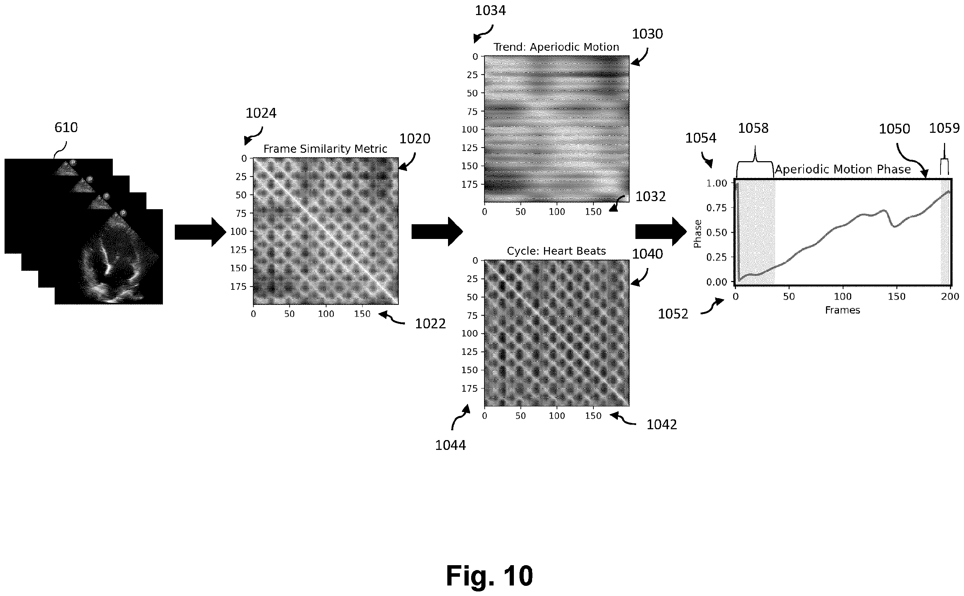

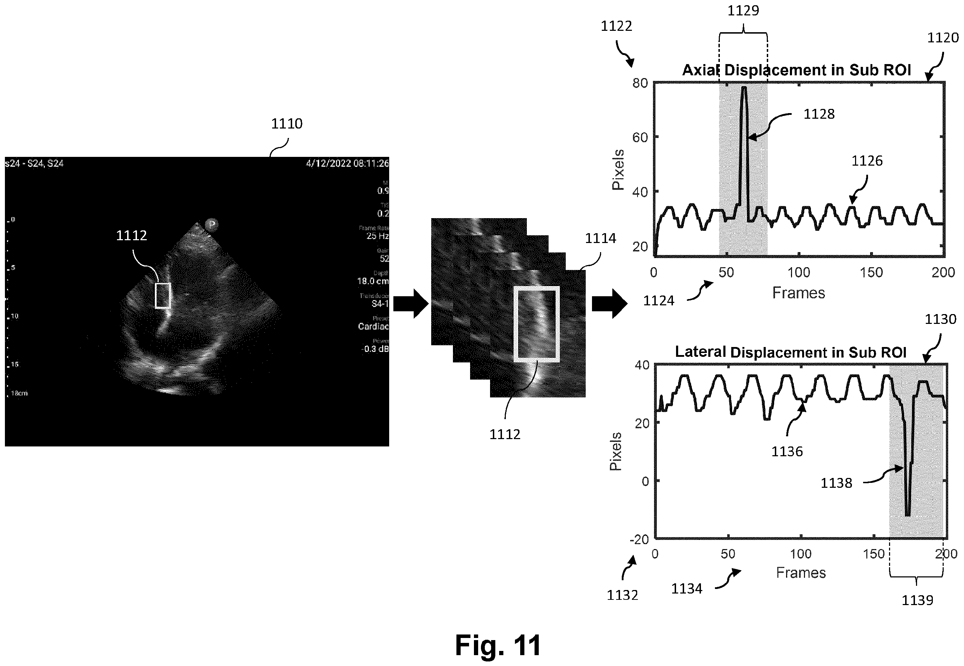

Step 504 of the method 500 includes extracting motion-corrupted ultrasound images. Motion-corrupted ultrasound images may be identified using any suitable technique, such as any techniques shown and described with reference to Figs. 10 and 11 hereafter. Once the motion-corrupted ultrasound images have been identified, they may be removed from the dataset so as to prevent them from interfering with the subsequent analysis, as described at step 506.

Step 506 of the method 500 includes forming temporal data blocks from the received ultrasound images. Temporal data blocks may be formed of contiguous non-motion-corrupted ultrasound image frames of at least one full periodic cycle. In that regard, temporal data blocks may be formed by removing ultrasound images frames that are motion corrupted or belonging to a periodic cycle which includes motion corrupted ultrasound images. In some aspects, the frames of a temporal data block may be selected such that they correspond to at least full cardiac beat cycle.

Step 508 of the method 500 includes determining whether the temporal block includes at least one periodic cycle. Periodic cycles may be identified using any suitable technique, such as by identifying peaks in the plot corresponding to a full cardiac beat cycle. If the temporal block does not include at least one periodic cycle, the method 500 may proceed to step 510.

Step 510 of the method 500 includes determining that not enough data was received at step 502. This may include outputting an indication to a graphical user interface indicating that not enough data was received at step 502. The method 500 may then revert to step 502 and additional ultrasound images may be received.

Step 512 of the method 500 includes performing quantification (or metric) of the ultrasound images of a temporal block. The processor of the system may be configured to perform any suitable quantification. For example, the processor may determine, at step 512, any of an end diastolic volume (EDV), an end systolic volume (ESV), stroke volume (SV), ejection fraction (EF), global longitudinal strain (GLS), or cardiac output (CO) for any ultrasound image frames, for example, for ultrasound image frames corresponding to at least one cardiac cycle, temporal data block, or all ultrasound image frames not extracted as corrupt at step 504. In some aspects, multiple temporal blocks may be created at step 506.

The steps 512-522 may be performed for each temporal block formed at step 506 or for all the temporal blocks as a whole. For example, the steps 512-522 may be performed simultaneously for all of the ultrasound images of all the temporal blocks formed at step 506. In some aspects, a quantification or metric may be calculated for each ultrasound image of the temporal blocks 506.

In some aspects, quantification of an ultrasound image at step 512 may additionally include determining an image quality of the ultrasound image. For example, determining an image quality may include calculating image quality factors such as endocardial border visualization, clarity of left ventricle, septal alignment, left ventricle depth coverage and foreshortening, temporal image quality, or any other features.

Step 514 of the method 500 includes outputting a graphical user interface. The graphical user interface may include an ultrasound image. The ultrasound image may be one of the ultrasound images received at step 502. In some aspects, the ultrasound image may be one of the ultrasound images of the temporal blocks formed at step 506. In some aspects, the ultrasound image may be an ultrasound image which was not extracted at step 504.

The graphical user interface may also include one or more quantifications or metrics such as any quantifications calculated at step 512. The graphical user interface may also include a plot of the metric for each ultrasound image of the temporal blocks. The plot may include indicators identifying regions of the plot corresponding to ultrasound images obtained as part of a full cardiac beat cycle which are not motion-corrupted. In some aspects, the plot may include indicators identifying regions of the plot corresponding to ultrasound images obtained that are not motion-corrupted but are not part of a full cardiac beat cycle. In some aspects, the plot may include indicators identifying regions of the plot corresponding to ultrasound images that are motion-corrupted.

Step 516 of the method 500 includes receiving a user input to accept the data, reject the data, or modify the data. The user input may be received through any suitable input device, such as a keyboard, a mouse, or a touchscreen.

Step 518 of the method 500 includes determining if the user input corresponds to the user accepting the data, the method 500 may progress to step 520, at which the method 500 ends. In some aspects, if the user input corresponds to the user rejecting the data, the method 500 may revert back to step 502 and additional ultrasound images may be received. If the user input corresponds to the user modifying the data, the method 500 may progress to step 522.

Step 522 of the method 500 includes receiving a user input modifying the data. The user input may be received through any suitable input device, and may include modifications to the quantifications or metrics calculated at step 512.

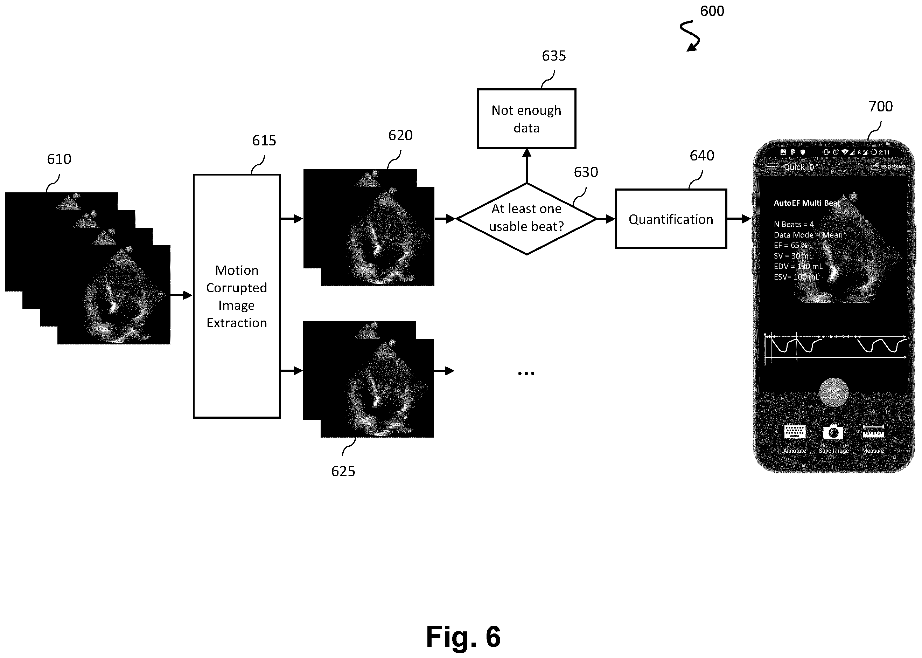

Fig. 6 is a graphical representation of a method 600 of extracting motion corrupted ultrasound images from a set of acquired ultrasound images and performing a quantification based on the acquired set, according to aspects of the present disclosure. As shown in Fig. 6, multiple ultrasound images 610 may be received by the system. The ultrasound images may be passed to a motion-corrupted image extraction module 615. The motion-corrupted image extraction module 615 may extract motion-corrupted images according to any of the methods or techniques described with reference to Figs. 10 and 11 hereafter. As shown in Fig. 6, the motion-corrupted image extraction module 615 may output several temporal data blocks. For example, a subset of ultrasound images 620 may form a first temporal data block. An additional subset of ultrasound images 625 may form a second temporal data block. In some aspects, the ultrasound images 620 of the first temporal data block may be acquired by the ultrasound image probe at a first a range of time prior to the time at which the second subset of ultrasound images 625 are obtained. In some aspects, a period of time may separate the time at which the first subset of ultrasound images 620 were obtained and the time at which the second subset of ultrasound images 625 were obtained.

In some aspects, after each temporal block is created, the ultrasound images of each temporal block may be analyzed to determine if it corresponds to at least one cardiac cycle. If, for any one temporal data block, the system determines that the temporal data block does not include enough ultrasound images to correspond to at least one cardiac cycle, the system may output an indication that there are not enough ultrasound images within a temporal data block to correspond to at least one cardiac cycle. In some aspects, the temporal data block which does not include enough ultrasound images may be discarded. The same process happens for each temporal data block, as shown in Fig. 6. After each temporal data block has been analyzed to ensure that it includes sufficient data, all of the temporal data blocks may be forwarded to a quantification module 640. The quantification module 640 may perform any suitable quantifications, such as any of those described with reference to step 512 of the method 500. After the quantification is complete, the system may generate a graphical user interface. The graphical user interface may be output to the display of a device 700.

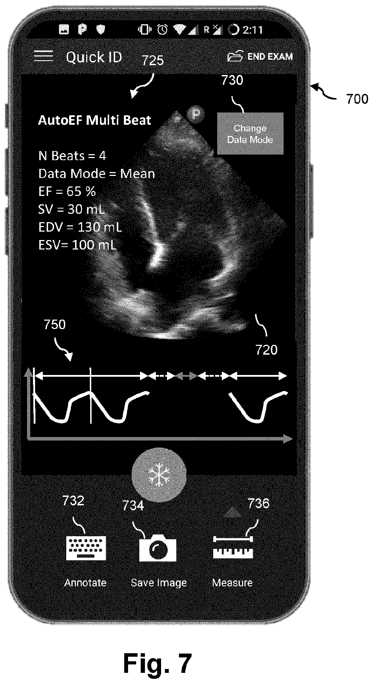

Fig. 7 is a graphical representation of a graphical user interface, according to aspects of the present disclosure. The graphical user interface may be displayed on the device 700. The device may be any kind of device including a smart phone, tablet, computer, laptop, portable medical devices, or any other suitable device. The graphical user interface includes a set of metrics 725.

The set of metrics 725 displayed on the graphical user interface may include any of the quantifications calculated at step 512. The set of metrics 725 may also include any other relevant information related to the ultrasound images, such as the time and date of the ultrasound, the patient's name and medical identification number, the ultrasound machine settings, and any other relevant information. These metrics may be any of the metrics determined at step 512 of the method 500.

In some aspects, the graphical user interface may include a 2D or 3D ultrasound image 720, which may be one of the ultrasound images received at step 502, one of the ultrasound images of the temporal blocks formed at step 506, or an ultrasound image that was not extracted at step 504. The ultrasound image 720 may be displayed alongside the set of metrics 725 to provide a visual representation of the ultrasound data. The ultrasound image 720 may also be annotated with graphical elements, such as arrows or text boxes, to highlight specific features or structures of interest.

The graphical user interface may indicate a number of beats used in the analysis reported under the N Beats metric. The metrics 725 may also include summary statistics. In some aspects, the user may select the "Change Data Mode" button 730 to alter the metric 725 to be any suitable other metrics. For example, the change data mode button 730 may toggle the metrics 725 between a default summary statistics mode and other types of modes. The default summary statistics mode may correspond to mean metrics over all beats analyzed. Some available options for other types of modes include median, mode, rank, or standard deviation. In some aspects, the user can optionally choose one of the N beats, and the graphical user interface may display metrics corresponding to the chosen beat alone. The metrics 725 may include any suitable types of metrics such as ejection fraction (EF), stroke volume (SV), global longitudinal strain (GLS), cardiac output (CO), or any other suitable metrics.

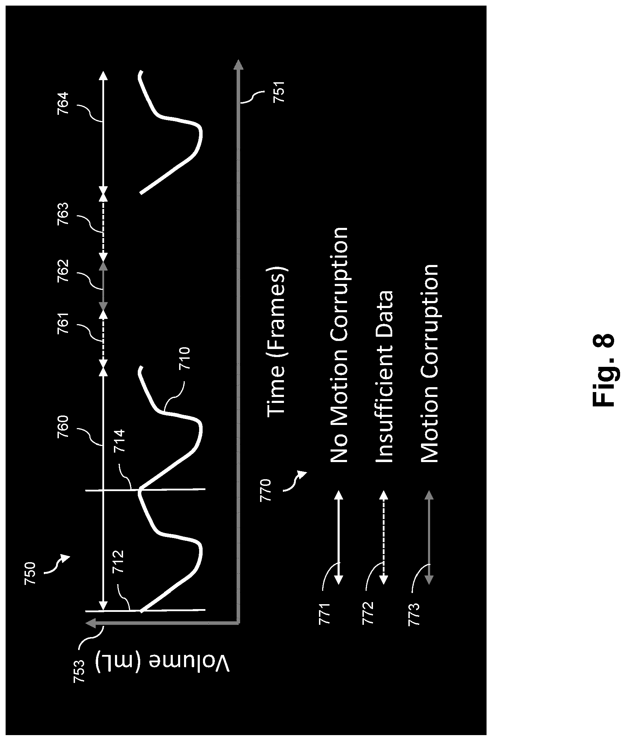

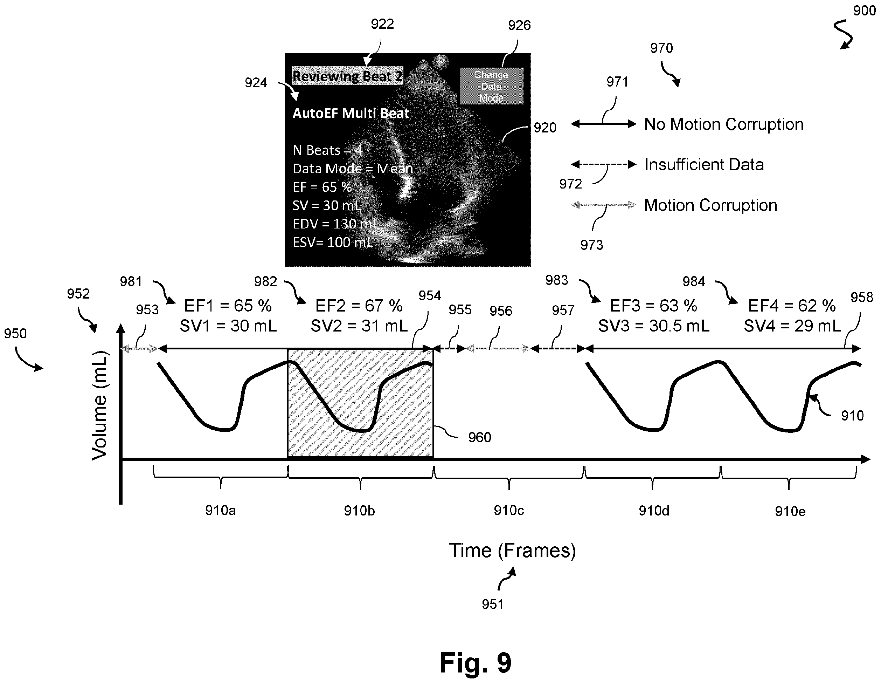

The graphical user interface also includes a plot 750. The plot 750 may show any of the metrics calculated over time, e.g., by frame. In some aspects, if the user chooses a specific beat, e.g., by the change data mode button 730 or selecting and/or moving various indicators within the plot 750. In some aspects, when a specific beat is selected, one ultrasound image corresponding to the selected beat may be displayed. In some aspects, all the ultrasound images corresponding to that beat will be highlighted in the plot 750 by an indicator (see e.g., indicator 960 of Fig. 9). The plot 750 will be described in more detail with reference to Fig. 8 below.

In some aspects, the graphical user interface includes an ultrasound image 720. The ultrasound image 720 may be any of the ultrasound images obtained at step 502 of the method 500 or any of the ultrasound images of the temporal data blocks formed at step 506. The ultrasound image 720 may be one of the ultrasound images corresponding to a selected periodic cycle, such as a cardiac beat cycle.

The graphical user interface also includes an annotation button 732, a save image button 734, and a measure button 736. In some aspects, a measurement indicator and scale may be presented to the user within the graphical user interface in response to the user selecting the measure button 736. In response to the user selecting the save image button 734, the ultrasound image 720 and/or metrics 725 may be stored in a memory of the device 700 or in another memory associated with the device 700. For example, the device 700 may be in wired or wireless communication with a storage device, such as an external memory or cloud storage server which may store the ultrasound image 720 and/or metrics 725. In some aspects, the graphical user interface may be modified to include various displays, buttons, or indicators allowing the user to provide notes associated with, or overlaid on, the ultrasound image 720. In some aspects, the graphical user interface may also be modified to allow the user to highlight, outline, or otherwise annotate the ultrasound image 720 and/or its corresponding features, metrics or indicators.

Fig. 8 is a graphical representation of a graphical user interface, according to aspects of the present disclosure. The graphical user interface may, in some instances, be a part or a region of the graphical user interface shown in Fig. 7.

The plot 750 may include an x-axis 751 corresponding to time. The time axis 751 may be in units of frames (e.g., ultrasound image frames). The y-axis 753 may correspond to volume in milliliters. However, the y-axis 753 may correspond to any of the metrics described with reference to step 512 of the method 500.

The plot 750 includes a dataset 710. The dataset 710 illustrates the metric, such as volume, over time and illustrates the periodic nature of the measured parameter. The plot 750 includes a number of indicators positioned over the data set 710. In some aspects, the system may categorize each ultrasound image frame based into one of three categories, shown by the key 770. For example, each ultrasound image frame of the ultrasound images received at step 502 of the method 500 may be categorized as an ultrasound image obtained that does not show motion corruption (e.g., the no motion corruption indicator 771), an ultrasound image obtained with motion corruption (e.g., the motion corruption indicator 773), or an ultrasound image obtained without motion corruption but within a periodic cycle in which all of the ultrasound images of the periodic cycle are not motion corrupted (e.g., some of the ultrasound images within the cycle are motion corrupted, shown by the insufficient data indicator 772). Indicators corresponding to each of these categories may be positioned within the plot 750 to reflect the data of the plot 750. For example, the first two periodic cycles of the data set 710 may correspond to ultrasound images obtained without motion corruption as shown by the indicator 760. The last periodic cycle shown within the plot 750 in Fig. 7 may be obtained without motion corruption as shown by the indicator 764. The ultrasound images obtained between the periodic cycles shown may not be usable. In some examples, the ultrasound images obtained between the periodic cycles shown may correspond to one periodic cycle, two periodic cycles, or more periodic cycles. However, some of these ultrasound image may have been motion-corrupted. This is shown by the indicator 762. In addition, while all of the ultrasound images between the periodic cycles shown in plot 750 may not have been motion corrupted, the remaining non-corrupted ultrasound images are a part of the same periodic cycle(s) as the images corresponding to the indicator 762. These ultrasound images may be identified by the indicators 761 and 762 and, while not being motion corrupted, are not part of a full uncorrupted periodic cycle, and therefore, may be discarded.

In some aspects, the indicators 771, 772, and 773 may be visually differentiated in any suitable way. For example, the indicator 771 may be green, the indicator 772 may be yellow, and the indicator 773 may be red. The indicators 771, 772, and 773 may be visually differentiated in any other way including using different colors, line patterns, widths, or varied by any other visual characteristic. In some aspects, any other types of indicators may be positioned within or adjacent to the different regions of the plot 750 to indicate the status of ultrasound images obtained at corresponding locations/times.

The plot 750 also includes two indicators 712 and 714. The indicators 712 and 714 may identify a single periodic cycle. In Fig. 8, the first periodic cycle may be identified by the indicators 712 and 714. In some examples, periodic cycles may be identified by any other indicator. For example, an indicator may include a box positioned around a periodic cycle or any other suitable method or indicator to highlight a periodic cycle. In addition, the indicators 712 and 714 and/or any other suitable similar indicators, may identify any suitable region of the data of the plot 750. For example, multiple periodic cycles may be identified and highlighted as well as portions of cycles or any other suitable regions or multiple regions.

Fig. 9 is a graphical representation of a graphical user interface 900, according to aspects of the present disclosure. The graphical user interface 900 includes an ultrasound image 920. The ultrasound image 920 may be similar to the ultrasound image 720. As shown in Fig. 9, the graphical user interface 900 may be displayed to a user to review specific periodic cycles, such as cardiac beats. For example, the ultrasound image 920 may be displayed along with a title 922. The title 922 may indicate which beat is currently displayed for review. In the example shown, beat 2 may be displayed for review. As explained in more detail hereafter, beat 2 may correspond to the region 910b.

In some aspects, the graphical user interface 900 may also include a set of metrics 924. The metrics 924 may include a type of the analysis performed, such as whether the analysis was performed on one beat or multiple beats. In some aspects, the metrics 924 may correspond to or be similar to the metrics 725 of Fig. 7.

The graphical user interface 900 may also include a key 970. The key 970 may be similar to the key 770 previously described. The key 970 includes three categories: an indicator 971 corresponding to no motion corruption, an indicator 973 corresponding to motion corruption, and an indicator 972 corresponding to insufficient data.

In some aspects, the graphical user interface 900 may include a plot 950. The plot 950 includes an x-axis 951 corresponding to time. The x-axis 951 may correspond to units of frames, such as ultrasound image frames, or any other unit of time. In some aspects, the plot 950 includes a y-axis 952 corresponding to a metric such as volume. In some aspects, the y-axis 952 may correspond to any other suitable metrics, such as any of the metrics quantified, for example, at step 512 of the method 500. The plot 950 includes a data set 910 corresponding to periodic cyclical measurements. Several periodic cycles, such as cardiac beats, are shown in the plot 950. For example, regions 910a, 910b, 910c, 910d, and 910e each may correspond to a cardiac beat.

The plot 950 may include several indicators corresponding to any of the three categories of the key 970. For example, an indicator 953 may be positioned over a portion of the plot 950. This may indicate to the user that the ultrasound images corresponding to this region of the plot 950 are motion-corrupted. As such, no data is present at this region, as it has been discarded. An indicator 954 is shown positioned over the curve 910 along the regions 910a and 910b. This may indicate to a user that the ultrasound images corresponding to the regions 910a and 910b are not motion-corrupted. An indicator 956 is shown over a portion of the region 910c. This may indicate to a user that the ultrasound images corresponding to the portion of the region 910c corresponding to the indicator 956 are motion corrupted. As a result, no data is shown in this portion of the region 910c. Indicators 955 and 957 are positioned on either side of the indicator 956. These indicators may indicate that there is insufficient data. In that regard, the ultrasound images obtained at the regions corresponding to the indicators 955 and 957 may not be motion corrupted. However, because they are in the same region 910c corresponding to a single cardiac beat, the ultrasound image frames obtained without motion corruption within the region 910c cannot be used because not all of the ultrasound images of the region 910c were obtained without motion corruption (e.g., the ultrasound images corresponding to the indicator 956 are motion corrupted and from the same cardiac beat corresponding to the region 910c). An indicator 958 is shown positioned over regions 910d and 910e. This indicator, like the indicator 954, may indicate that the ultrasound images of the regions 910d and 910e are not motion corrupted and the data is displayed.

In some aspects, as shown in Fig. 8, metrics may be calculated and displayed for each usable beat of the plot 950. For example, the plot 950 includes metrics 981 corresponding to the ejection fraction and stroke volume corresponding to the first cardiac beat of region 910a. These metrics may be calculated based only on the ultrasound images of the first cardiac beat of the region 910a. Similarly, the plot 950 also includes metrics 982 corresponding to the ejection fraction and stroke volume corresponding to the second cardiac beat of region 910b. The plot 950 also includes metrics 983 corresponding to the ejection fraction and stroke volume corresponding to the third cardiac beat of region 910d. The plot 950 also includes metrics 984 corresponding to the ejection fraction and stroke volume corresponding to the fourth cardiac beat of region 910e. As shown, in some instances, no metrics may be displayed above the cardiac beat corresponding to the region 910c because these ultrasound image frames were discarded. In some aspects, any suitable metrics may be calculated and displayed for each cardiac beat as shown, including any of the metrics described herein.

In some aspects, Fig. 9 may illustrate an aspect of the disclosure in which the user has the ability to interact with the graphical user interface to accept, review or reject metrics. In some aspects, the user may be able to review and revise quantification of the multi beat results. As shown in Fig. 9, the graphical user interface may also include an indicator 960. The indicator 960 may identify the specific beat being analyzed. For example, as shown by the title 922 in the example graphical user interface 900, the metrics of beat 2 corresponding to region 910b identified by the indicator 960 within the plot 950 may be displayed. The user may review the data and results for a specific beat by adjusting indicator 960 which may also be referred to as a beat selection slider. In some aspects, the user may move the indicator 960 to review other beats. Similarly, the user may adjust the size of the indicator 960 by moving the edges of the indicator 960 to review other regions of the data. In some aspects, the user may also select different data statistics for display.

In some aspects, the user may be able to modify contours within individual ultrasound image frames within the chosen beat. The results will update based on the updated contours and the graphical user interface will display the updated results. In some aspects, the user may delete a beat from multi beat analysis. Users can choose a specific beat to review using the indicator 960, review the beat in terms of contour tracking and quantitative results and then can delete the beat if it is not optimal. In some aspects, the user may revise the results associated with one specific beat, multiple beats, or all analyzed beats.

Fig. 10 is a graphical representation of a method of extracting motion corrupted ultrasound images, according to aspects of the present disclosure. In some aspects, Fig. 10 may correspond to a method performed by an aperiodic motion extraction block, such as the motion-corrupted image extraction module 615 of Fig. 6.

The purpose of the aperiodic motion extraction block may be to identify frames corrupted by aperiodic motion such as probe motion or patient breathing. This block may be designed according to image and signal processing methods reported in either of U.S. Patent No. 6,589,917 to Jago et al. issued on July 8, 2003 and the IEEE publication entitled "Image-Based Methods for Phase Estimation, Gating, and Temporal Superresolution of Cardiac Ultrasound" by D.R. Chittajallu et al., vol. 66, no. 1, published January 2019, both of which are incorporated by reference in their entirety.

In some aspects, the processor of the system may use a cardiac and respiratory phase estimation approach to identify motion-corrupted ultrasound image frames. In some aspects, the plurality of ultrasound images 610 may be received by the processor. The plurality of ultrasound images 610 may form a cine loop. A first step of the process may include generating a frame similarity matrix 1020. In some aspects, normalized cross-correlation is calculated for all pairs of frames present inside the loops resulting the symmetric frame similarity matrix 1020 with dimensions $N \times N$ where $N$ is the number of frames in the cine loop. This may be illustrated by the axis 1022 and the axis 1024 each corresponding to the number of image frames of the cine loop. Each row of this metric can be considered as a univariate time series containing both cardiac and aperiodic motion. Next, a trend extraction method, such as a Hodrick Prescott ("HP") filter may be applied to each row to separate the periodic signature corresponding to cardiac and aperiodic motion. In some aspects, the HP filter decomposes a time series as a sum of two components including (1) a low frequency trend component corresponding to the aperiodic motion which is used to form the aperiodic motion matrix 1030 and a high frequency seasonality component corresponding to the cardiac beats to form the heartbeat matrix 1040. As shown in Fig. 10, each of the aperiodic motion matrix 1030 and heartbeat matrix 1040 may each include axes of the same length of the axes of the matrix 1020. In some aspects, the matrix 1030 may include an axis 1032 and an axis 1034 may be of a length corresponding to the number of ultrasound images 610. Similarly, the matrix 1040 may include an axis 1042 and an axis 1044 may be of a length corresponding to the number of ultrasound images 610.

As shown in Fig. 10, a periodogram may be calculated for each row of the heartbeat matrix 1040. In some aspects, entropy of spectral density may be estimated and the row with the smallest entropy may be chosen for aperiodic motion extraction and the corresponding time series from the aperiodic motion matrix 1030 may be extracted from frame number determination. In addition, instantaneous phase may be extracted from the chosen time series using a transform resulting in the aperiodic motion phase curve 1050 shown in Fig. 10. The curve 1050 may include an x-axis 1052 corresponding to frame number and including the total number of frames of the ultrasound images 610. The curve 1050 may also include a y-axis corresponding to phase. The highlighted regions 1058 and 1059 of the phase curve 1050 may correspond to aperiodic motion. In the example of Fig. 10, the most aperiodic motion occurs in the frames located around the local minima of the phase curves which corresponds to the sharp changes from maximum to minimum phase values (1 to 0). To identify these frames, phase values around the local minima can be used as a filter. The following equation may be used to identify frames corrupted by aperiodic motion:

$$Frame_{discard} = \{frame\ number \mid Phase_{motion}^{aperiodic} < w \lor Phase_{motion}^{aperiodic} > (1 - w)$$

where, $w = 0.2$. In some aspects, $w$ may be empirically chosen.

Fig. 11 is a graphical representation of a method of identifying motion corrupted ultrasound images, according to aspects of the present disclosure. In some aspects, Fig. 11 may describe an additional or alternative method of identifying ultrasound images with aperiodic and/or non-cardiac motion.

For example, another approach for aperiodic motion detection may include the use of speckle tracking employed in speckle tracking echocardiography for strain estimation. In some aspects, a sub region-of-interest 1112 containing bright speckle content from the myocardium may be chosen for speckle tracking. The selection process of the sub region-of-interest 1112 may be automated using deep learning object detection methods such as Mask R-CNN, YOLO, or any other suitable deep learning method. In some aspects, a fixed search region 1114 around the center of the sub region-of-interest 1112 may be provided as an input. This input may be provided by the user or as an output of the deep learning method. In some aspects, this input may be provided to the tracking algorithm. In this application, the search region 1114 may be set to a fixed number of pixels in both axial and lateral dimensions. In some aspects, the interframe trajectory of the sub region-of-interest 1112 may be tracked over the entire cine loop and a median filter may be applied to the estimated trajectory. In some aspects, the trajectory is a vector containing axial and lateral components. For example, an axial displacement component may be sufficient to identify frames with aperiodic motion corruption. In other examples, a lateral displacement component may be sufficient to identify frames with aperiodic motion corruption. The axial and lateral displacement components may be shown in the axial displacement graph 1120 and the lateral displacement graph 1130 respectively.

In that regard, the axial displacement graph 1120 may include an x-axis 1124 corresponding to frame number and a y-axis 1122 corresponding to pixels. In that regard, the axial displacement of the sub region-of-interest 1112 within the ultrasound images may be tracked and shown within the graph 1120 by the dataset 1126. As shown, the bulk of the dataset 1126 corresponds to relatively uniform periodic motion. However, a spike 1128 in the dataset 1126 illustrates how aperiodic motion of the sub region-of-interest 1112 as shown highlighted by the region 1129.

Similarly, the lateral displacement graph 1120 may include an x-axis 1134 corresponding to frame number and a y-axis 1132 corresponding to pixels. In that regard, the lateral displacement of the sub region-of-interest 1112 within the ultrasound images may be tracked and shown within the graph 1130 by the dataset 1136. As shown, the bulk of the dataset 1136 corresponds to relatively uniform periodic motion. However, a spike 1138 in the dataset 1136 illustrates how aperiodic motion of the sub region-of-interest 1112 as shown highlighted by the region 1130.

In some aspects, the processor may implement various combinations of the method described with reference to Fig. 10 as well as the method described with reference to Fig. 11. For example, an estimate of aperiodic motion may be extracted using the method of Fig. 10. Then, frames identified to have aperiodic motion can be tracked using the approach of Fig. 11. Frames identified to have aperiodic motion by both approaches can be then discarded from analysis.

In an additional aspect, ultrasound image frames with aperiodic motion may be identified based on accelerometer data of the ultrasound probe in an IMU data stream stored in conjunction of the cine loop. Frames corrupted by probe motion may be identified using the IMU data stream. The results from IMU and image-based data analysis may then be combined to identify frames corrupted by aperiodic motion.

In an additional aspect, ultrasound image frames with aperiodic motion may be identified based on motion compensated normalized cross-correlation between original and motion compensated cine loop. For example, accumulated Lagrangian displacement fields may be estimated using speckle tracking. All frames with the cine loops may then be warped to the first frame assuming the first reference as a temporal frame of reference. Then, inter frame NCC may be estimated between the first frame and all the warped frames. The frames corresponding to low motion compensated NCC may be classified as frames corrupted by aperiodic motion. In that regard, aperiodic motion may result in peak hop errors in the estimated displacement fields resulting in inaccurate motion compensation and low NCC values.

In an additional aspect, ultrasound image frames with aperiodic motion may be identified using a deep learning model. The input of the deep learning model may be the stored cine loop and the output may be the frame numbers corrupted by motion. During training, expert annotation may be performed on a frame level to identify frames with and without motion corruption. Then, deep learning classification models such as ResNet, EfficientNet, Inceptionv3, or any other suitable deep learning model can be trained to provide frame level predictions. Each frame may be classified into two categories such as corrupted or non-corrupted. Finally, using the frame level classification from the deep learning model, the cine loop can be divided into optimal temporal data blocks for quantification analysis.

Persons skilled in the art will recognize that the apparatus, systems, and methods described above can be modified in various ways. Accordingly, persons of ordinary skill in the art will appreciate that the aspects encompassed by the present disclosure are not limited to the particular exemplary aspects described above. In that regard, although illustrative aspects have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound imaging system (100), comprising:

a processor (134) configured for communication with a display (132), a transducer array (112) of a handheld ultrasound probe (110), and a memory (138), wherein the processor is configured to:

receive a plurality of ultrasound images (610) corresponding to a view of a patient anatomy;
identify one or more motion corrupted ultrasound images of the plurality of ultrasound images;
extract the one or more motion corrupted ultrasound images from the plurality of ultrasound images;
form a first temporal data block, the first temporal data block comprising at least a subset (620) of the remaining ultrasound images from the plurality of ultrasound images;
determine whether the first temporal data block corresponds to at least one periodic cycle;
calculate a metric (725, 924) based on the first temporal data block; and
generate an output for the display, the output compirsing:

an ultrasound image (720, 920) of the plurality of ultrasound images; the metric;
a first status indicator (771, 971) corresponding to the first temporal data block; and
a second status indicator (773, 973) corresponding to the extracted motion corrupted ultrasound images.

2. The ultrasound imaging system of claim 1, wherein the processor (134) is further configured to determine an image quality of the plurality of ultrasound images (610).

3. The ultrasound imaging system of claim 1 or 2, wherein the processor (134) is further configured to identify the one or more motion corrupted images by identifying and tracking a feature within the plurality of ultrasound images.

4. The ultrasound imaging system of claim 3, wherein, to track the feature, the processor (134) is configured to:

determine a change in location of the feature within each ultrasound image of the plurality of ultrasound images (610); and
identify an ultrasound image in which the change in location of the feature exceeds a threshold.

5. The ultrasound imaging system of any of claims 1 to 4, wherein the processor (134) is further configured to identify the one or more motion corrupted images based on one or more of traditional image processing, RF data peak hopping, AI image processing, AI processing on RF data, or AI processing on ultrasound B-mode data.

6. The ultrasound imaging system of any of claims 1 to 5, wherein the metric comprises at least one of an end diastolic volume (EDV), end systolic volume (ESV), stroke volume (SV), ejection fraction (EF), global longitudinal strain (GLS), or cardiac output (CO).

7. The ultrasound imaging system of any of claims 1 to 6, wherein the periodic cycle comprises a cardiac beat cycle.

8. The ultrasound imaging system of any of claims 1 to 7, wherein the metric comprises an average.

9. The ultrasound imaging system of any of claims 1 to 7,

wherein the first temporal data block comprises a first subset (620) of the remaining ultrasound images from the plurality of ultrasound images; and
wherein the processor (134) is further configured to form a second temporal data block, the second temporal data block comprising a second subset (625) of the remaining ultrasound images from the plurality of ultrasound images,
wherein the first temporal data block and the second temporal data block are temporally spaced by the motion corrupted ultrasound images.

10. The ultrasound imaging system of claim 9, wherein the metric comprises an average of data from the first temporal data block and the second temporal data block.

11. The ultrasound imaging system of any of claims 1 to 10, wherein the processor (134) is further configured to receive an input from the user accepting the metric or rejecting the metric.

12. The ultrasound imaging system of any of claims 1 to 11, wherein the processor (134) is further configured to receive a first user input identifying a first periodic cycle of the first temporal data block.

13. The ultrasound imaging system of claim 12, wherein the processor (134) is further configured to discard a subset of ultrasound images corresponding to the first periodic cycle of the first temporal data block based on a second user input.

14. A method (500) for extracting motion corrupted ultrasound images from a set of acquired ultrasound images and performing a quantification based on the acquired set, comprising:

   receiving (502) a plurality of ultrasound images (610) from an ultrasound imaging system (100), the plurality of ultrasound images corresponding to a view of a patient anatomy;
   identifying one or more motion corrupted ultrasound images of the plurality of ultrasound images;
   extracting (504) the one or more motion corrupted ultrasound images from the plurality of ultrasound images;
   forming (506) a first temporal data block, the first temporal data block comprising at least a subset (620) of the remaining ultrasound images from the plurality of ultrasound images;
   determining (508) whether the first temporal data block corresponds to at least one periodic cycle;
   calculating (512) a metric based on the first temporal data block; and
   generating (514) an output, the output compirsing:

      an ultrasound image (720, 920) of the plurality of ultrasound images;
      the metric (725, 924);
      a first status indicator (771, 971) corresponding to the first temporal data block; and
      a second status indicator (773, 973) corresponding to the extracted motion corrupted ultrasound images.

15. A computer program product comprising computer-readable instructions which, when executed by a processor, cause the processor to carry out the method of claim 14.

**Fig. 1**

EP 4 516 238 A1

Processor Circuit 210

Processor 260

MEMORY 264

Instructions 266

Communication Module 268

**Fig. 2**

**Fig. 3**

EP 4 516 238 A1

**Fig. 4**

EP 4 516 238 A1

**Fig. 5**

**Fig. 6**

**Fig. 7**

EP 4 516 238 A1

Fig. 8

EP 4 516 238 A1

Fig. 9

**Fig. 10**

EP 4 516 238 A1

**Fig. 11**

EP 4 516 238 A1

Europäisches
Patentamt

European
Patent Office

Office européen
des brevets

# EUROPEAN SEARCH REPORT

**Application Number**

EP 23 19 5358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/273669 A1 (SCHNEIDER ROBERT JOSEPH [US]) 28 September 2017 (2017-09-28) | 1,2,5, 7-15 | INV. A61B8/08 |
| Y | * abstract * | 3,4 | A61B8/00 |
| A | * figures 1-6 *<br>* paragraph [0007] – paragraph [0053] * | 6 | |
| Y | US 2005/033123 A1 (GARDNER EDWARD A [US] ET AL) 10 February 2005 (2005-02-10)<br>* abstract *<br>* figures 1-5 *<br>* paragraph [0003] – paragraph [0046] * | 3,4 | |
| A | OEZDEMIR IPEK ET AL: "Improved quantitative contrast-enhanced ultrasound imaging of hepatocellular carcinoma response to transarterial chemoembolization",<br>2019 IEEE 16TH INTERNATIONAL SYMPOSIUM ON BIOMEDICAL IMAGING (ISBI 2019), IEEE,<br>8 April 2019 (2019-04-08), pages 1737-1740, XP033576415,<br>DOI: 10.1109/ISBI.2019.8759238<br>[retrieved on 2019-07-10]<br>* abstract *<br>* figures 1-6 *<br>* Sections I- IV * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 27 December 2023 | Moehrs, Sascha |

EPO FORM 1503 03.82 (P04C01)

**EP 4 516 238 A1**

## ANNEX TO THE EUROPEAN SEARCH REPORT
## ON EUROPEAN PATENT APPLICATION NO.

EP 23 19 5358

27-12-2023

| Patent document cited in search report | | | Publication date | Patent family member(s) | | | Publication date |
|---|---|---|---|---|---|---|---|
| US 2017273669 | A1 | | 28-09-2017 | CN | 107072635 | A | 18-08-2017 |
| | | | | EP | 3192053 | A1 | 19-07-2017 |
| | | | | JP | 6535088 | B2 | 26-06-2019 |
| | | | | JP | 2017526467 | A | 14-09-2017 |
| | | | | US | 2017273669 | A1 | 28-09-2017 |
| | | | | WO | 2016038491 | A1 | 17-03-2016 |
| US 2005033123 | A1 | | 10-02-2005 | US | 2005033123 | A1 | 10-02-2005 |
| | | | | US | 2009010511 | A1 | 08-01-2009 |
| | | | | US | 2009016586 | A1 | 15-01-2009 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 6589917 B, Jago

**Non-patent literature cited in the description**

• **D.R. CHITTAJALLU et al.** *Image-Based Methods for Phase Estimation, Gating, and Temporal Super-resolution of Cardiac Ultrasound*, January 2019, vol. 66 (1)